# EUROPÄISCHE PATENTSCHRIFT

(11) **EP 2 216 056 B1**
(45) Veröffentlichungstag und Bekanntmachung des Hinweises auf die Patenterteilung: **22.08.2018**
(21) Anmeldenummer: 10163587.8
(22) Anmeldetag: 26.08.2003
(51) Int. Cl.: A61L 29/16, A61L 31/16

(54) **Medizinische Vorrichtung zur Arzneimittelabgabe**
medical device for dispensing medicaments
dispositif medical pour l'administration de medicaments

(30) Priorität: 20.09.2002 DE 10244847
(43) Veröffentlichungstag der Anmeldung: 11.08.2010
(62) Teilanmeldung aus: 07017424.8
(73) Patentinhaber: Bayer Intellectual Property GmbH, 40789 Monheim (DE)
(72) Erfinder: Speck, Ulrich, 13465, Berlin (DE); Scheller, Bruno, D-66399 Mandelbachtal (DE)
(74) Vertreter: Zimmermann & Partner Patentanwälte mbB

(56) Entgegenhaltungen:
- WO-A-00/44414
- WO-A-00/45744
- WO-A-92/11890
- WO-A-92/15282
- WO-A1-2004/006976
- WO-A2-02/076509
- DE-A1- 10 115 740
- DE-C1- 4 225 553
- US-A- 5 370 614
- US-A1- 2002 123 505

## Beschreibung

Die Erfindung betrifft eine medizinische Vorrichtung zur Arzneimittelabgabe für die selektive Therapie bestimmter Gewebeabschnitte oder Organteile und ein Verfahren zur Herstellung derartiger arzneimittelbeschichteter Geräte.

Zahlreiche Erkrankungen betreffen nicht den gesamten Organismus gleichzeitig sondern sind auf bestimmte Gewebearten, häufig auch auf sehr begrenzte einzelne Gewebebezirke oder Organteile beschränkt. Beispiele dafür finden sich unter den Tumor-, Gelenk- und Gefäßerkrankungen.

Die Pharmakotherapie auch dieser Erkrankungen erfolgt im allgemeinen durch orale oder intravenöse Gabe von Arzneistoffen, die sich im ganzen Körper verteilen und in vielen Fällen gerade bei schweren Erkrankungen unerwünschte Wirkungen in gesunden Geweben und Organen verursachen, die die therapeutische Anwendung begrenzen. Eine selektive Therapie der erkrankten Gewebe wurde mittels spezifisch an erkranktes Gewebe bindende Arzneistoffe (z. B. Antikörper) unter Beibehaltung des Applikationsweges oder durch selektive Verabreichung, z.B. durch direkte Injektion in das erkrankte Gewebe oder durch Zufuhr über Katheter zu den das erkrankte Gewebe versorgenden Blutgefäßen, erreicht. Im Falle der selektiven Verabreichung entstehen durch die meist kurze Wirkdauer der Arzneistoffe und die invasiven Verabreichungswege Probleme, da sich eine beliebig wiederholte Gabe verbietet. Bei selektiver Verabreichung über den das erkrankte Gewebe versorgenden Blutstrom ergibt sich das zusätzliche Problem ungenügender Extraktion der Arzneistoffe bei der raschen Passage des Blutes oder der Wirkstofflösung durch die Blutgefäße.

Diesen Problemen wurde bisher durch unterschiedliche pharmazeutische Präparate mit verzögerter Wirkstofffreigäbe, arzneimittelfreisetzende Implantate oder für längere Zeit funktionsfähige selektive Zugangswege wie implantierte Katheter etc. begegnet.

Es ist bereits bekannt, die Oberfläche von in den Körper eingebrachten medizinischen Geräten, insbesondere Kathetern, mit Mitteln zur Verbesserung der Gleitfähigkeit oder zur Verhinderung der Blutgerinnung, jedoch ohne therapeutische Wirkung, zu beschichten.

Darüber hinaus werden Katheter mit speziellen Vorrichtungen versehen, um Arzneimittel in die Arterienwand zu injizieren, beispielsweise mittels Nadeln oder hohem Injektionsdruck über eine an der Gefäßwand anliegende Perforation der Katheterwand.

Andere Prinzipien beruhen darauf, die Kontaktzeit zwischen Arterienwand und einer über den Katheter applizierten Wirkstoffzubereitung zu verlängern, indem entweder der Blutstrom für einen entsprechenden Zeitraum unterbunden wird, z. B. Doppelballonkatheter mit zwischen den Ballons befindlicher mit der Arzneistofflösung gefüllter Kammer oder Hohlräumen zwischen der z.B. mit Wülsten versehenen Ballonaußenwand, wobei der Blutstrom durch einen den Ballon passierenden Kanal in begrenztem Maße aufrechterhalten werden kann.

Gemäß der US 5 102 402 werden Arzneistoffe in Form von Mikrokapseln zur verzögerten Wirkstofffreigabe lose in vorgeformten Vertiefungen von Ballonkathetern untergebracht. Nach Expansion des Ballons sollen die Mikrokapseln in die Gefäßwand gedrückt werden, dort verbleiben und den oder die Wirkstoffe langsam freisetzen. Zahlreiche Autoren schlagen auch vor, Arzneistoffe in Hydrogel eingebettet auf Ballonkatheter aufzubringen, wobei die Funktion des Hydrogels als Haftmittel, zur Verbesserung der Gleitfähigkeit oder Verzögerung der Freisetzung der Arzneistoffe offen bleibt.

Nachteil der genannten Produkte ist in jedem Falle der komplexe Aufbau mit entsprechenden Problemen bei Herstellung, Qualitätskontrolle und Kosten sowie zusätzlichen, Arzt und Patient belastenden Arbeitsschritten bei der Anwendung. Ein Teil der genannten Methoden führt zu einer über die beabsichtigte Gefäßerweiterung hinausgehenden gänzlich unerwünschten Gefäßverletzung. Andererseits hat jede zur Verlängerung der Kontaktzeit vorgesehene Maßnahme eine zusätzliche Minderversorgung der nachgeschalteten Gewebe mit Blut und Sauerstoff zur Folge.

Der Vollständigkeit halber wird noch auf eine in der WO 01/24866 beschriebenen Vorrichtung zum Verhindern der Restenose verwiesen, die mit einer von natürlichen Zellmembranen abgeleiteten lipiden Ceramide-Substanz beschichtet sind. Diese Substanz wird aufgrund ihrer bei gebräuchlichen Arzneistoffen nicht anzutreffenden Affinität zu den Zellwänden der Arterienwand verwendet. In der Fachliteratur wird jedoch weiterhin die Auffassung vertreten, dass eine Arzneimittelprophylaxe der Restenose eine Freisetzung der erforderlichen Wirkstoffe über Tage erfordert.

Dokument US 5,370,614 betrifft ein Verfahren zum Herstellen eines Ballonkatheters zur Arzneimittelabgabe. Der Ballonkatheter beinhaltet ein Hülle, die den Ballon umgibt, wobei die Hülle eine Längsschwächungslinie aufweist, und ein Arzneimittel enthaltendes viskoses Matrixmaterial zwischen dem Ballon und der Hülle, so dass, wenn der Ballon angeordnet und in einem Körperlumen inflatiert ist, die Hülle an der Schwächungslinie reißt und das viskose Matrixmaterial auf das Körperlumen freisetzt.

Dokument WO 00/44414 A1 betrifft gleitfähige medizinische Geräte, die darin eingebettet physiologisch aktive Inhaltstoffe aufweisen. Verschiedene Polymersubstrate, wie zum Beispiel Katheter, Stents, Dilatationsballone, Führungsdrähte, Endotrachealtuben, Instrumente, Implantate und andere medizinische Vorrichtungen können Gleitfähigkeit und Abriebfestigkeit aufweisen, wie auch ein im Wesentlichen konstantes Freisetzungsprofil der physiologisch aktiven Inhaltsstoffe über längere Perioden.

Der Erfindung liegt die Aufgabe zugrunde, eine Vorrichtung für eine auf bestimmte Gewebebereiche oder Organteile begrenzte Arzneimittelabgabe bereitzustellen, die ohne schädigenden Einfluss auf gesundes Gewebe eine starke therapeutische Wirkung ausübt, den Patienten nur wenig belastet und mit geringem Aufwand angewendet und hergestellt werden kann.

Erfindungsgemäß wird die Aufgabe mit einer gemäß den Merkmalen der Ansprüche unabhängigen ausgebildeten bzw. hergestellten Vorrichtung gelöst. Aus den Unteransprüchen ergeben sich weitere Merkmale und vorteilhafte Weiterbildungen der Erfindung.

Durch die Erfindung werden in einem einfachen Herstellungsverfahren verbesserte arzneistofftragende Ballonkatheter bereitgestellt, die vielseitig anwendbar sind und eine sofortige Wirkstofffreigabe ermöglichen. Überraschend und entgegen der Lehrmeinung ist keine andauernde Wirkstofffreisetzung aus einer inerten Matrix (Polymer, Hydrogel, Mikrokapseln etc.) oder speziellen chemischen oder physikalischen Zuständen der Wirkstoffe erforderlich oder nützlich. Daher werden auch keine aufwendigen Techniken zur Produktion oder Kontrolle von Depotformulierungen benötigt.

Die Beschichtung von auf Kathetern befindlichen Ballonen mit Arzneistoffen gemäß der vorliegenden Erfindung ist insofern von besonderem Nutzen als nach dem Erweitern von Blutgefäßen oder anderen Hohlräumen im Körper mit den Ballonen häufig der Bedarf an therapeutischen Maßnahmen besteht, um eine Verengung oder einen Verschluss des mit dem Ballon unter Druck geschaffenen Lumens zu verhindern, Tumorwachstum zu begrenzen oder Heilungsprozesse einschließlich der Bildung von Kollateralkreisläufen zu fördern. Dies kann durch Arzneistoffe erreicht werden, die ihre Wirkung in unmittelbarer Nähe der Ballonoberfläche entfalten. Die Arzneistoffe haften auf dem Weg zum Ziel - meist durch intensiv durchblutete Arterien - bis zur Entfaltung des Ballons fest auf diesem, werden dann während der kurzen, oft nur Sekunden andauernden Kontaktzeit des entfalteten Ballons an das Gewebe in wirksamer Dosis abgegeben und von diesem in einer Weise aufgenommen, die das Abspülen durch den nach Deflation des Ballons sofort wieder einsetzenden Blutstrom vermeidet.

Zur Beschichtung sind Drähte wie sie zur Führung von Kathetern verwendet werden, Nadeln und Katheter bzw. Teile von Kathetern, die zumindest für kurze Zeit mit Druck gegen erkrankte Gewebe gepresst werden, vorgesehen. Bevorzugte Kathetermaterialien sind Polyamide, Polyamid-Gemische und Copolymere, Polyethylenterephthalat, Polyethylen und Copolymere, Polyurethan, Naturkautschuk und dessen Derivate. Die Länge und der Durchmesser der für die Pharmakotherapie vorgesehenen Bereiche der Katheter bzw. Ballone ist für die Anwendung nicht von entscheidender Bedeutung, da die Dosierung in *µ*g Wirkstoff/ mm² Oberfläche berechnet wird. Beispielsweise sind für die Koronardilatationen Ballone im Bereich von 2 - 4 mm Durchmesser und von 1,0-4,0 cm Länge gebräuchlich. Für andere Gefäße können auch Ballone bis zu > 20 mm Durchmesser und Längen bis zu > 10 cm eingesetzt werden. Die zu beschichtenden Oberflächen können glatt (d.h. ohne besondere Struktur zur Aufnahme der Wirkstoffe), aufgeraut oder in beliebiger Weise mit Strukturen versehen sein, wobei spezielle Oberflächenstrukturen nicht Voraussetzung für die Haftung der Wirkstoffe sind, die Haftung aber auch nicht behindern. Die Haftung der Wirkstoffe auf den Ballonoberflächen wird ausschließlich durch die Wahl geeigneter Lösungsmittel und ggf. die Haftung beeinflussende Zusatzstoffe bewirkt. Sie ist selbst auf äußerlich vollständig glatten Ballonoberflächen überraschend fest.

Alle Oberflächen können zusätzlich mit Substanzen beschichtet worden sein oder werden, die die Gleitfähigkeit der Produkte verbessern, die Gerinnung von Blut an der Oberfläche verhindern oder sonstige Eigenschaften der Medizinprodukte verbessern, ohne dass die zur Beschichtung benutzten Materialien an die Umgebung abgegeben werden müssen und ohne dass die Beschichtung die Abgabe der Wirkstoffe zur Behandlung der Zielgewebe und damit die Wirksamkeit wesentlich einschränkt.

Ballonkatheter werden aus sehr dünnen Kunststoffschläuchen durch Aufweitung eine Segments von 1 bis ca. 10 cm Länge geformt. Die aufgeweitete, sehr dünnwandige Ballonmembran wird anschließend in mehrere längs zur Katheterachse angeordnete Falten gelegt und fest um die Katheterachse gewickelt, so dass der aufgeweitete Bereich im gefalteten Zustand einen nur minimal größeren Durchmesser aufweist als der übrige Katheter. Die enge Faltung der Ballonhülle ist Voraussetzung für die problemlose Passage des Ballonkatheters durch Einführungsschleusen, Führungskatheter und z.B. stark verengte Abschnitte von Blutgefäßen.

Die Ballone von Kathetern können in gefaltetem und entfaltetem Zustand beschichtet werden, wobei in jedem Fall eine intakte, ausreichend gleichmäßige Beschichtung der Oberfläche erzielt wird und die Wirkstoffe auch während des Einfaltens eines im entfalteten Zustand beschichteten Ballonkatheters ausreichend fest an dessen Oberfläche haften.

Die Herstellung eines in entfaltetem Zustand beschichteten Ballons erfolgt ohne Beeinträchtigung der Beschichtung beispielsweise durch die Verwendung von Ballonhüllen mit vorgeformten Falten und Biegungen, deren Struktur im Material durch das Aufdehnen nicht verloren geht und die nach Ablassen des Druckes aus dem Ballon bewirken, dass sich die Ballonhülle wieder regelrecht wenigstens lose einfaltet, ohne dass es einer äußeren Kraft als primäre Ursache bedarf. Erst danach werden die vorgeformten Falten von außen oder durch Vakuum zusammengepresst. In keinem Falle sind Falten zum Festhalten des Wirkstoffs erforderlich. Weiterhin kann die Einfaltung durch geringe mechanische Kräfte mittels sehr glatter Materialien bewirkt werden, wobei die Werkzeuge auch beispielsweise mit schlüpfrigen biokompatiblen Flüssigkeiten benetzt sein können, in denen sich die Wirkstoffe nicht oder jedenfalls nicht gut lösen.

Gemäß einer weiteren Erfindungsvariante werden die Ballone fertig gefalteter Ballonkatheter durch Tauchen in niedrig viskose Wirkstofflösungen beschichtet. Dabei dringen Lösungsmittel und Wirkstoff zwischen die extrem engen Falten und bilden dort einen überraschend gleichmäßigen und in der Dosis reproduzierbaren Belag, der durch keinen weiteren Arbeitsschritt beschädigt wird. Die außen anhaftende Lösung bzw. der nach Trocknen des Lösungsmittels außen anhaftende Belag kann dort belassen oder in einem weiteren Arbeitsschritt entfernt werden, so dass nur der von den Falten des Ballons verdeckte Wirkstoff erhalten bleibt.

Nach der Beschichtung kann bei gefaltetem Ballon ein Stent auf den Ballonkatheter geschoben und auf diesem festgepresst werden. Danach ist nur noch die Sterilisation, z. B. mittels Ethylenoxid, erforderlich.

Der so gestaltete Arbeitsgang ist außerordentlich einfach, wenig störanfällig und auch mit mechanisch, chemisch und physikalisch empfindlichen Beschichtungsmaterialien durchzuführen. Es hat sich gezeigt, dass die Beschichtung nach diesem Verfahren zu keiner unerwünschten Lockerung oder Verklebung der Faltung führt und dass der derart aufgetragene Wirkstoff fest genug haftet, um auf dem Weg durch das Blut nicht abgetragen zu werden, andererseits bei Inflation des Ballons im Zielgewebe den Wirkstoff weitgehend freigibt.

Als Arzneistoffe kommen stark lipophile, weitgehend wasserunlösliche, an beliebige Gewebebestandteile bindende stark wirksame Arzneistoffe in Frage. Als lipophil werden Arzneistoffe bezeichnet, deren Verteilungskoeffizient Butanol: wässriger Puffer pH 7 = 0.5, bevorzugt = 1 und besonders bevorzugt = 5 bzw. Octanol: wässriger Puffer pH 7 = 1, bevorzugt = 10, besonders bevorzugt > 50 ist. Alternativ oder zusätzlich sollen die Arzneistoffe zu > 10 %, bevorzugt zu > 50 %, besonders bevorzugt zu > 80 % reversibel und/oder irreversibel an Zellbestandteile binden. Bevorzugt sind Substanzen zur Hemmung der Zellproliferation oder auch entzündlicher Prozesse oder Antioxidantien wie Paclitaxel und andere Taxane, Rapamycin und verwandte Substanzen, Tacrolimus und verwandte Substanzen, Corticoide, Sexualhormone (Östrogen, Estradiol, Antiandrogene) und verwandte Substanzen, Statine, Epothilone, Probucol, Prostacycline, Angiogeneseinduktoren etc.
Erfindungsgemäß wird Paclitaxel eingesetzt.
Die Substanzen liegen bevorzugt als trockene Festsubstanz oder als Öl auf den Oberflächen der unterschiedlichen Medizinprodukte vor. Bevorzugt sind Partikel geringster Größe (in der Mehrzahl < 5 *µ*m, bevorzugt < 1 *µ*m, besonders bevorzugt < 0.1 *µ*m), besonders bevorzugt sind amorphe, nicht kristalline Strukturen feinster Partikelgröße, die bei Kontakt mit Gewebe wegen ihrer großen Oberfläche trotz grundsätzlich geringer Wasserlöslichkeit der Arzneistoffe rasch in Lösung gehen und nicht als Mikrokapseln fungieren, d. h. sich spontan und schnell lösen. Dabei ist es ausreichend, dass eine wirksame Dosis in Form kleinster oder amorpher Partikel vorliegt; größere Partikel tragen zwar zur Wirkstoffkonzentration im Gewebe kaum bei, stören aber auch nicht. Die Dosierung richtet sich nach der erwünschten Wirkung und der Wirksamkeit des verwendeten Arzneistoffes. Sie kann bis zu 5 *µ*g/ mm² erreichen, wobei dies keine Obergrenze darstellt. Geringere Dosierungen sind leichter zu realisieren.
Weiter ist hierin beschrieben, dass eine gute Haftung an den Oberflächen der Katheter, Nadeln oder Drähte bei Verbesserung der Aufnahme in die Gewebe wird durch Einbetten von stark lipophilen, schlecht wasserlöslichen Wirkstoffen in eine leicht wasserlösliche Matrixsubstanz erreicht wird. Als Matrixsubstanzen sind niedermoleklare (Molekulargewicht < 5000 D, bevorzugt < 2000 D) hydrophile Substanzen wie in vivo verwendete Kontrastmittel und Farbstoffe für unterschiedliche diagnostische Verfahren in der Medizin, Zucker und verwandte Substanzen wie Zuckeralkohole, niedermolekulare Folyethylenglycole, biokompatible organische und anorganische Salze wie z. B. Benzoate, Salze und andere Derivate der Salicylsäure etc. geeignet. Als Kontrastmittel sei auf die jodierten Röntgenkontrastmittel und die paramagnetischen Chelate verwiesen, Beispiele für Farbstoffe sind Indocyaningrün, Fluorescein und Methylenblau, Hilfsstoffe können auch zur Verbesserung der Lagerfähigkeit der Produkte dienen, spezielle ergänzende pharmakologische Wirkungen verursachen oder der Qualitätskontrolle dienen.
Weiter ist hierin beschrieben, dass die pharmazeutischen "Wirkstoffe an Partikel adsorbiert oder mit niedermolekularer Matrix auf die Oberflächen geeigneter Medizinprodukte aufgebracht werden können. Als Partikel sind wiederum als biokompatibel bekannte Diagnostika wie Ferrite und diverse Kontrastmittel für die Sonographie geeignet.

Hilfsstoffe aller Art können in geringerer oder höherer Dosis als die Wirkstoffe eingesetzt werden.

Die Beschichtung der Medizinprodukte erfolgt mittels Lösungen, Suspensionen oder Emulsionen der genannten. Arzneistoffe und Hilfsstoffe. Geeignete Lösungs- Suspendier-oder Emulsionsmedien sind beispielsweise Ethanol, Isopropanol, Ethylacetat, Diethylether, Aceton, Dimethylsulfoxid, Dimethylformamid, Glycerin, Wasser oder Mischungen derselben. Die Auswahl der Lösungsmittel folgt entsprechend der Löslichkeit der Wirkstoffe und Zusatzstoffe sowie der Benetzung der zu beschichtenden Oberflächen und der Wirkung auf die Struktur der nach Verdampfen der Lösungamittel zurückbleibenden Beschichtung und Partikel, deren Haftung auf der Oberfläche und die Wirkstoffübertragung in das Gewebe während sehr kurzer Kontaktzeiten. Die Auftragung kann beispielsweise durch Tauchen, Bestreichen, Auftragen mittels Volumenmesseinrichtungen oder Besprühen jeweils bei unterschiedlichen Temperaturen und ggf. Dampfsättigungen der Lösungsmittel in der Atmosphäre geschehen. Der Vorgang kann mehrfach, ggf. auch unter Verwendung verschiedener Lösungsmittel und Hilfsstoffe, wiederholt werden.

Die Ballone fertig gefalteter Ballonkatheter lassen sich durch Tauchen in wirkstoffhaltige Lösungen oder andere Maßnahmen erstaunlich gleichmäßig, reproduzierbar, in der Dosis steuerbar und ohne Beeinträchtigung der Funktion der Katheter beschichten. Bei wiederholtem Tauchen in ungesättigten Wirkstofflösungen kommt es wider Erwarten nicht zu einer vollständigen Ablösung des vorher aufgetragenen Wirkstoffs sondern zu einer reproduzierbaren Erhöhung des Wirkstoffgehaltes der Ballone.

Überschüssige Lösung bzw. überschüssige, außen lose anhaftende Substanzen aus der Beschichtungslösung können mit einfachen Methoden entfernt werden, ohne dass es zu einer Beeinträchtigung der Wirksamkeit der Beschichtung kommt.

Die erfindungsgemäß ausgebildeten und hergestellten medizinischen Vorrichtungen kommen kurzzeitig, d. h. für Sekunden, Minuten oder wenige Stunden mit dem Gewebe in Kontakt. In einigen Fällen ist es erwünscht, das Gewebe in unmittelbarer Nähe des Medizinproduktes pharmakologisch zu behandeln, zum Beispiel an überschießendem Wachstum als Reaktion auf eine Verletzung zu hindern oder Tumorwachstum zu reduzieren oder die Einsprossung von Blutgefäßen zu fördern oder Entrzündungsreaktionen zu vermindern. In all diesen Fällen kann mit dem oben beschriebenen Verfahren eine hohe lokale Arzneistoffkonzentration für erstaunlich lange zeit erzielt werden. Ein wesentlicher Vorteil ist die außerordentliche Vielfalt der Einsatzmöglichkeiten der beschriebenen Produkte und Verfahren.

Eine bevorzugte Anwendung ist die Verminderung der durch die Gefäßdilatation mittels Ballonkathetern induzierte Hyperproliferation der Gefäßwände. Diese ist im Bereich gegebenenfalls implantierter Gefäßstützen (Stents) auch durch Beschichtung der Stents mit Arzneistoffen zu erzielen, allerdings nur im unmittelbar durch den Stent bedeckten Gefäßbereich. Die beschichteten Ballonkatheter behandeln darüber hinaus die behandlungsbedürftigen Bereiche kurz vor und kurz hinter dem Stent, sie können ohne erneute Stentimplantation den Bereich innerhalb bereits vorhandener Stents behandeln oder Gefäße, in die kein Stent implantiert werden soll oder kann. Vorteilhaft gegenüber den über einen langen Zeitraum Arzneistoff freisetzenden Stents ist die bessere Einheilung bei gleichzeitig guter Hemmung der Hyperproliferation und das geringere Thromboserisiko.

Nachfolgend werden mehrere Ausführungsformen der Erfindung am Beispiel der Beschichtung von Ballonkathetern sowie in Bezug auf die Haftung der Beschichtung im Blut, die Restenosehemmung und den Wirkstoffgehalt der Katheter beschrieben.

### Beispiel 1:

### Beschichten eines expandierten Ballonkatheters mit Paclitaxel in Ethylacetat

Ballonkatheter der Fa. BMT, Oberpfaffenhofen/ München, Deutschland, mit der Bezeichnung Joker Lite, Ballongröße 2.5 mm x 20 mm, werden nach maximaler Expansion für 1 min. über die gesamte Ballonlänge in Ethylacetat, 18.8 mg Paclitaxel/ ml, + 1% pharmazeutisches Olivenöl, getaucht, getrocknet:
Paclitaxelgehalt 39 *µ*g (nach Extraktion mit Ethanol, HPLC).

### Beispiel 2:

### Beschichten eines gefalteten Ballonkatheters mit Paclitaxel in Ethylacetat

Ballonkatheter der Fa. BMT, Oberpfaffenhofen/ München, Deutschland, mit der Bezeichnung Joker Lite, Ballongröße 2.5 mm x 20 mm, werden im gefalteten Zustand für 1 min. über die gesamte Ballonlänge in Ethylacetat, 18.8 mg Paclitaxel/ ml, + 1% pharmazeutisches Olivenöl, getaucht, getrocknet:
Paclitaxelgehalt 69 *µ*g.

### Beispiel 3:

### Beschichten eines gefalteten Ballonkatheters mit Paclitaxel in Ethylacetat

a) Ballonkatheter der Fa. BMT, Oberpfaffenhofen/ München, Deutschland mit der Bezeichnung Joker Lite, Ballongröße 2.5 mm x 20 mm, werden im gefalteten zustand für 1 min. über die gesamte Ballonlänge in Ethylacetat, 16.6 mg Paclitaxel/ ml, getaucht, 4 h getrocknet: Paclitaxelgehalt 54 *µ*g
b) Ebenso, jedoch noch 2 mal 5 sec. mit 1 h Trocknungszeit nach jedem Tauchvorgang in Lösung A (= 3.33 ml Ethylacetat + 100.0 mg Paclitaxel) getaucht: Paclitaxelgehalt 126 *µ*g
c) Ebenso, jedoch noch 4 mal 5 sec. mit 1 h Trocknungszeit nach jedem Tauchvorgang in die gleiche Lösung getaucht: Paclitaxelgehalt 158 *µ*g

### Beispiel 4:

### Beschichten eines Ballonkatheters mit Paclitaxel in Aceton

350 mg Paclitaxel in 9.0 ml Aceton lösen; Ballonkatheter der Fa. BMT, Oberpfaffenhofen/ München, Deutschland mit der Bezeichnung Joker Lite, Ballongröße 2.5 mm x 20 mm, werden im maximal expandierten Zustand für 1 min. über die gesamte Ballonlänge getaucht, entnommen, das Lösungsmittel wird bei Raumtemperatur 12 h getrocknet. Danach wird der Ballon deflatiert und mit PTFE-beschichtetem Werkzeug in üblicher Weise eingefaltet. Optional kann ein Stent geeigneter Größe auf den Ballon gecrimmt werden: 29 *µ*g Paclitaxel auf dem Ballon.

### Beispiel 5:

### Beschichten eines Ballonkatheters mit Paclitaxel in Aceton

a) Tauchen gefaltete Ballonkatheter der Fa. BMT mit der Bezeichnung Allegro, Ballongröße 2.5 x 20 mm in ein Gemisch von 0.15 ml Ethanol + 4.5 *µ*l Ultravist 300 ( Röntgenkontrastmittel der Schering AG, Berlin, Deutschland) + 1.35 ml Aceton + 0.8 mg Sudanrot + 30.0 mg Paclitaxel:
   Die gefalteten Ballonabschnitte der Katheter werden 5 x getaucht, das 1. Mal für 1 Minute, dann 3 h Trocknungszeit, danach 4 mal für je 5 sec im Abstand von 1 h; danach wurde ein Stent aufgecrimpt und der Katheter mit Stent in üblicher Weise mit Ethylenoxid sterilisiert: Paclitaxelgehalt 172 *µ*g, keine mittels HPLC detektierbaren Zersetzungsprodukte des Wirkstoffs
b) Statt Ultravist 300 wird eine gesättigte wässrige Mannit-Lösung zugesetzt
c) Statt Ultravist 300 wird eine gesättigte wässrige Natrium-Salicylat Lösung, pH 7.5, zugesetzt.
d) Der fertigen Lösung nach (5a) werden 5 mg Acetylsalicylsäure zugesetzt.
e) Der fertigen Lösung nach (5a) werden 5 mg Glycerin zugesetzt

### Beispiel 6:

### Haftung des Wirkstoffs in Blut

Es wurden 12 Ballonkatheter der Fa. BMT mit der Bezeichnung Allegro, Ballongröße 2.5 x 20 mm, verwendet. Je 6 Stück der gefalteten Ballonabschnitte der Katheter wurden entweder in [0.15 ml Ethanol + 4.5 *µ*l Ultravist 300 + 1.35 ml Aceton + 0.8 mg Sudanrot+ 30.0 mg Paclitaxel] oder in [1.5 ml Ethylacetat + 0.8 mg Sudanrot + 31.0 mg Paclitaxel] 5 x getaucht, das 1. Mal für 1 Minute, dann 3 h Trocknungszeit, danach 4 mal für je 5 sec im Abstand von 1 h; danach wurden je 3 gefaltete Ballone jeder Serie in 50 ml Humanblut 5 min bei 37 °C leicht bewegt und dann zur Analyse des Paclitaxel-Gehaltes entnommen: Verminderung der Mittelwerte (n=3 je Beschichtungsmethode) durch 5 Minuten Bewegung in Blut im Vergleich zu je 3 Kontrollkathetern, die nicht in Blut inkubiert wurden.

| | |
|---|---|
| Aceton: | 12 % |
| Ethylacetat: | 10 % |

### Beispiel 7:

### Untersuchung der Restenosehemmung nach Angioplastie und Stentimplantation an den Koronarien von Schweinen

Gefaltete Ballonkatheter der Fa. BMT Typ Joker Lite, BMT 3.5 x 20 mm oder 3.0 x 20 mm wurden entweder in
Lösung A) 3.33 ml Ethylacetat (EA)+ 100.0 mg Paclitaxel oder in
Lösung B) 0.45 ml Ethanol + 100 *µ*l Ultravist-370 + 4.5 ml Aceton (Ac) + 150.0 mg Paclitaxel
für 1 min eingetaucht und bei Raumtemperatur über Nacht getrocknet. Ein weiterer (niedrige Dosis = L) bzw. 4 weitere (hohe Dosis = H) Tauchvorgänge wurden jeweils nur für 5 Sekunden am nächsten Tag im Abstand von 1 h durchgeführt.
Wirkstoffgehalt nach 2 maligem Tauchen in Lösung (B) im Mittel 250 *µ*g, bei 5 maligem Tauchen in Lösung (B) 500*µ*g, in Lösung (A) 400 *µ*g.
Insgesamt 22 Schweinen wurde mittels der mit Paclitaxel beschichteten Katheter oder mittels unbeschichteter Katheter Stents in die linke Vorderwand- oder Seitenwand-Koronararterie implantiert, wobei die Gefäße zur Stimulation der Restenose durch Gewebehyperplasie leicht überdehnt wurden. Nach 5 Wochen wurden die Tiere reangiographiert und das Maß der Gefäßverengung auf den Angiogrammen mit einem automatischen Computerprogramm gemessen.

| Gruppe | Stenose (%) |
|---|---|
| Unbeschichtet | 50.49 |
| AcL | 20.22 |
| EAH | 36.01 |
| AcH | 0.86 |
| p | .004 |

Quantitative Koronarangiographie 5 Wochen nach Stentimplantation mit unbeschichteten und beschichteten Kathetern; Stenose = prozentuale Verminderung des Lumendurchmessers im Bereich des Stents im Vergleich zu dem Lumendurchmesser unmittelbar nach Stentimplantation Mittelwert und statistische Signifikanz des Behandlungseffekts.

### Beispiel 8:

### Wirkstoffgehalt der Katheter nach Gefäßdilatation und Stentimplantation

Die Ballone aus Beispiel 8 wurden nach Stentimplantation und Entnahme aus den Tieren auf ca. 3 cm Länge von den Ballonkathetern abgetrennt und in 1.5 ml Ethanol überführt. Der Paclitaxelgehalt wurde mittels HPLC bestimmt. Alle verfügbaren beschichteten Ballone und eine Auswahl unbeschichteter Ballone wurden untersucht.
Koronar,

| | | | |
|---|---|---|---|
| 3.0 x 20 mm, Beschichtung: | Ac hoch | 38 ± 4 *µ*g | (n=4) |
| | Ac niedrig | 22 ± 5 *µ*g | (n=2) |
| | EEE hoch | 41 | (n=1) |
| 3.5 x 20 mm, Beschichtung: | Ac hoch | 37 ±10 *µ*g | (n=8) |
| | Ac niedrig | 26 ± 6 *µ*g | (n=8) |
| | EEE hoch | 53 ± 9 *µ*g | (n=9) |
| Unbeschichtet (unabhängig von Größe und Gefäßgebiet) | | 0.9 ± 1.0 *µ*g | (n=7) |

Aus Beispiel 6 ergibt sich, dass maximal 10 % der Dosis verloren gehen bevor der Ballon expandiert wird und ca. 10 % der Dosis auf dem Ballon verbleiben.

### Vergleichs-

### Beispiel 9:

Probucol wird in einer Konzentration von 100 mg/ ml in Aceton eingebracht; die Lösung wird wie in den vorhergehenden Beispielen beschrieben zum Beschichten der Ballonkatheter eingesetzt.

### Vergleichs-

### Beispiel 10:

Rapamycin wird in einer Konzentration von 10 mg/ ml in Diethylether gelöst. Die Beschichtung der Ballonanteile der Katheter erfolgt wie in den vorhergehenden Beispielen beschrieben; die Ballone sollen nach der Entnahme aus der Beschichtungslösung möglichst sofort horizontal ausgerichtet und ständig um ihre Längsachse gedreht werden.

### Vergleichs-

### Beispiel 11:

Epothilon B wird in einer Konzentration von 2 mg/ ml in Ethylacetat gelöst; die Lösung wird wie in den vorhergehenden Beispielen beschrieben zum Beschichten der Ballonkatheter eingesetzt.

## Patentansprüche

1. Verfahren zur Herstellung eines Ballonkatheters, auf welchem ein Wirkstoff haftet, wobei das Verfahren die folgenden Schritte umfasst:
a) Bereitstellen eines Ballonkatheters umfassend einen Ballon;
b) Bereitstellen einer Lösung bestehend aus Paclitaxel und einem Lösungsmittel;
c) Auftragen der Lösung auf die Oberfläche des Ballons; und
d) Trocknen des Ballons.

2. Verfahren nach Anspruch 1, **dadurch gekennzeichnet, dass** die Lösung aus Paclitaxel und einem Lösungsmittel ausgewählt aus Ethylacetat, Aceton, Ethanol, Glycerin oder Mischungen davon besteht.

3. Verfahren nach Anspruch 1 oder 2, **dadurch gekennzeichnet, dass** die Lösung aus Paclitaxel und einem Lösungsmittel ausgewählt aus Ethylacetat und Aceton besteht.

4. Verfahren nach einem der vorherigen Ansprüche, **dadurch gekennzeichnet, dass** die Lösung durch Tauchen, Streichen, Sprühen oder mittels Volumenmesseinrichtung auf die Oberfläche des Ballons aufgebracht werden, wobei überschüssige Medien und lose an der Oberfläche haftende Substanzen entfernt werden.

5. Verfahren nach Anspruch 4, **dadurch gekennzeichnet, dass** der Beschichtungsvorgang bei reproduzierbarer Erhöhung des Wirkstoffgehalts mit gleichen oder verschiedenen Lösungsmitteln mehrfach durchgeführt wird.

6. Verfahren nach einem der vorherigen Ansprüche, **dadurch gekennzeichnet, dass** als Wirkstoffträger gefaltete Ballone vorgesehen sind, die vor oder nach der Sterilisation ohne oder mit aufgebrachtem Stent beschichtet werden.

7. Verfahren nach Anspruch 6, **dadurch gekennzeichnet, dass** die Ballone in entfaltetem Zustand mit Paclitaxel beschichtet werden und das Falten der Ballone mit gleitfähigem, ggf. mit biokompatiblen Gleitmedien benetztem Werkzeug durchgeführt wird.

8. Verfahren nach Anspruch 4, **dadurch gekennzeichnet, dass** mit einem Ballonkatheter verbundene Stents vor oder nach einem Beschichtungsvorgang montiert werden.

9. Verfahren nach Anspruch 4, **dadurch gekennzeichnet, dass** der fertig beschichtete Ballonkatheter mittels Ethylenoxid sterilisiert wird.

10. Verfahren nach einem der vorherigen Ansprüche, wobei der Ballonkatheter eine medizinische Vorrichtung zur Arzneimittelabgabe für die selektive Therapie bestimmter erkrankter Gewebeabschnitte oder Organteile ist, wobei an der Oberfläche des zumindest kurzzeitig das erkrankte Gewebe unter Druck kontaktierenden Ballons das lipophile, weitgehend wasserunlösliche an beliebige Gewebebestandteile bindende Paclitaxel mit nach Gewebekontakt sofortiger Wirkstofffreigabe haftet.

11. Verwendung des gemäß einem der vorherigen Ansprüche hergestellten Ballonkatheters zur Herstellung einer medizinischen Vorrichtung für die Behandlung von Gefäßerkrankungen oder Durchblutungsstörungen.

12. Verwendung des gemäß einem der Ansprüche 1 bis 10 hergestellten Ballonkatheters zur Herstellung einer medizinischen Vorrichtung für die Schaffung von offenen Passagen im Körper.

## Claims

1. Process for the manufacture of a balloon catheter, on which a drug adheres, wherein the process comprises the following steps:
a) providing a balloon catheter comprising a balloon;
b) providing a solution consisting of paclitaxel and a solvent;
c) applying the solution onto the surface of the balloon; and
d) drying the balloon.

2. Process according to claim 1, **characterized in that** the solution consists of paclitaxel and a solvent selected from ethyl acetate, acetone, ethanol, glycerol or mixtures thereof.

3. Process according to claim 1 or 2, **characterized in that** the solution consists of paclitaxel and a solvent selected from ethyl acetate and acetone.

4. Process according to any of the preceding claims, **characterized in that** the solution is applied to the surface of the balloon by dipping, painting or spraying or using a volume measuring device, wherein excessive medium and substances loosely adhering to the surface will be removed.

5. Process according to claim 4, **characterized in that** the coating process is repeatedly conducted with a reproducible increase of the drug content with the same or different solvents.

6. Process according to any of the preceding claims, **characterized in that** folded balloons are provided as drug carriers, which are coated before or after sterilization with or without a mounted stent.

7. Process according to claim 6, **characterized in that** the balloons are coated with the paclitaxel in the unfolded state, and that the folding of the balloons is conducted with a slidable tool, possibly wetted with a biocompatible friction media.

8. Process according to claim 4, **characterized in that** stents connected with a balloon catheter are mounted before or after the coating process.

9. Process according to claim 4, **characterized in that** the readily coated device is sterilized by ethylene oxide.

10. Process according to any of the preceding claims, wherein the balloon catheter is a medical device for delivery of medication for the selective therapy of particular diseased tissue sections or organ parts, wherein the lipophilic, largely water insoluble paclitaxel that binds to any tissue parts adheres to the surface of the balloon, that contacts diseased tissue at least shortly under pressure, with immediate release of the drug upon tissue contact.

11. Use of the balloon catheter manufactured according to any of the preceding claims for manufacturing a medical device for treating vasculature diseases or circulatory disorders.

12. Use of the balloon catheter manufactured according to any of claims 1 to 10 for manufacturing a medical device for creating patent passages in the body.

## Revendications

1. Procédé de fabrication d'un cathéter à ballonnet auquel adhère une substance active, le procédé comprenant les étapes suivantes :
a) fourniture d'un cathéter à ballonnet comprenant un ballonnet ;
b) fourniture d'une solution constituée de paclitaxel et d'un solvant ;
c) application de la solution sur la surface du ballonnet ; et
d) séchage du ballonnet.

2. Procédé selon la revendication 1, **caractérisé en ce que** la solution est constituée de paclitaxel et d'un solvant choisi parmi l'acétate d'éthyle, l'acétone, l'éthanol, le glycérol ou les mélanges de ceux-ci.

3. Procédé selon la revendication 1 ou 2, **caractérisé en ce que** la solution est constituée de paclitaxel et d'un solvant choisi parmi l'acétate d'éthyle et l'acétone.

4. Procédé selon l'une des revendications précédentes, **caractérisé en ce que** la solution est appliquée sur la surface du ballonnet par trempage, par enduction, par pulvérisation ou au moyen d'un dispositif volumétrique, avec élimination des fluides en excès et des substances qui adhèrent d'une manière lâche à la surface.

5. Procédé selon la revendication 4, **caractérisé en ce que** l'opération de revêtement est mise en oeuvre à plusieurs reprises, avec des solvants identiques ou différents, pour une augmentation reproductible de la teneur en substance active.

6. Procédé selon l'une des revendications précédentes, **caractérisé en ce qu'**on prévoit en tant que support de substance active des ballonnets repliés, qui sont revêtus avant ou après la stérilisation, avec ou sans un stent appliqué.

7. Procédé selon la revendication 6, **caractérisé en ce que** les ballonnets sont revêtus de paclitaxel à l'état déplié, le repliement des ballonnets étant mis en oeuvre avec un outil lubrifié, éventuellement mouillé par des fluides lubrifiants biocompatibles.

8. Procédé selon la revendication 4, **caractérisé en ce que** des stents, reliés à un cathéter à ballonnet, sont montés avant ou après une opération de revêtement.

9. Procédé selon la revendication 4, **caractérisé en ce que** le cathéter à ballonnet ayant reçu un revêtement final est stérilisé à l'oxyde d'éthylène.

10. Procédé selon l'une des revendications précédentes, dans lequel le cathéter à ballonnet est un dispositif médical pour l'administration de médicaments pour la thérapie sélective de certains segments tissulaires ou parties d'organes malades, le paclitaxel lipophile, sensiblement insoluble dans l'eau et se fixant à des constituants tissulaires quelconques, adhérant, avec une libération de la substance active immédiate après contact avec le tissu, à la surface du ballonnet qui, au moins pendant un bref laps de temps, est en contact sous pression avec le tissu malade.

11. Utilisation du cathéter à ballonnet fabriqué selon l'une des revendications précédentes, pour la fabrication d'un dispositif médical pour le traitement de maladies vasculaires ou de troubles circulatoires.

12. Utilisation du cathéter à ballonnet fabriqué selon l'une des revendications 1 à 10 pour la fabrication d'un dispositif médical destiné à créer des passages ouverts dans le corps.
